# FASCICULE DE BREVET EUROPEEN

(11) **EP 2 255 179 B1**
(45) Date de publication et mention de la délivrance du brevet: **11.07.2018**
(21) Numéro de dépôt: 09722131.1
(22) Date de dépôt: 18.03.2009
(51) Int. Cl.: G01N 27/90

(54) **DISPOSITIF DE SURVEILLANCE DE LA STRUCTURE D'UN VEHICULE**
VORRICHTUNG ZUR ÜBERWACHUNG DER STRUKTUR EINES FAHRZEUGS
DEVICE FOR MONITORING THE STRUCTURE OF A VEHICLE

(30) Priorité: 20.03.2008 FR 0851819
(43) Date de publication de la demande: 01.12.2010
(73) Titulaire: AIRBUS (SAS), 31700 Blagnac (FR)
(72) Inventeur: MOREAU, Katell, F-75015 Paris (FR); ROUET, Vincent, F-78630 Orgeval (FR); ROLET, Sébastien, F-31830 Plaisance du Touch (FR)
(74) Mandataire: Gicquel, Olivier Yves Gérard
(86) Numéro de dépôt international: PCT/EP2009/002005
(87) Numéro de publication internationale: WO 2009/115315

(56) Documents cités:
- EP-A- 1 136 820
- EP-A- 1 783 501
- FR-A- 2 874 689
- FR-A- 2 884 605
- US-A1- 2002 163 333
- Specialist Contributors: "Instrumentation reference Book - Third Edition" In: "Instrumentation reference Book - Third Edition", 1 January 2003 (2003-01-01), Elsevier, XP055382304,

## Description

La présente invention a pour objet de suivre l'état de structures métalliques soumises à des contraintes mécaniques, vibratoires ou et environnementales. Elle met en oeuvre une méthodologie dans un système électronique embarqué autonome permettant la détection de criques dans ces structures métalliques, en temps réel. L'invention s'applique plus particulièrement aux domaines des transports, aériens, maritimes et terrestres.

Les structures des moyens de transport, dans un cas particulier les avions, sont soumises à des contraintes mécaniques, environnementales ou et vibratoires lors de leurs utilisations. Ces structures sont notamment régulièrement inspectées pour évaluer des fissures naissantes: une inspection rapide est effectué à chaque escale, et une autre, plus approfondie, est réalisée lors de maintenances dites de type C-check, après 2400 h de vol. Ces dernières sont longues et coûteuses car il est nécessaire de démonter des panneaux d'accès pour vérifier l'état des structures métalliques de l'avion, inaccessibles en temps normal. Réalisées périodiquement, ces inspections ne permettent donc pas un suivi continu des criques et de leurs évolutions. Il en est de même, dans le domaine ferroviaire, de certaines parties de trains, notamment de trains à grande vitesse.

Des systèmes de suivi des structures sont développés pour optimiser et réduire ces maintenances et par ce fait, leurs coûts et leurs durées. Par exemple des capteurs à courant de Foucault sont utilisés pour détecter des fissures sur des structures métalliques comme l'illustrent les documents US-A-6 952 095, GB-A-2 400 445, GB-A-2 396 427.

Pour interpréter les informations issues de ces capteurs, plusieurs interfaces électroniques ont été réalisées. L'une d'entre elles interprète l'impédance, plus particulièrement la phase, issue du capteur. Cette interface est utilisée pour former une image en deux dimensions de la surface à inspecter, comme le décrit le document US-A-5 006 800. Les inconvénients de cette méthode sont son encombrement et sa forte consommation électrique. De ce fait, la méthode ne peut être embarquée, et les résultats ne sont pas donnés en temps réel.

D'autres interfaces interprètent une conductivité issue d'un capteur et sont également utilisées pendant les maintenances. Le suivi des criques ne peut, par conséquent, être continu.

D'autres systèmes, utilisant des capteurs acoustiques, ont aussi été mis au point pour étudier, de façon continue, l'état des structures, comme le décrit le brevet WO 2006 111679. Ces méthodes sont souvent très consommatrices en énergie. Les équipements doivent être, par conséquent, branchés sur le réseau électrique embarqué dans l'avion. Ces équipements sont en outre très encombrants.

Il existe enfin des systèmes embarqués qui comptabilisent le nombre de contraintes reçues par l'avion, comme décrit dans le document EP-A-1 018 641. Ces contraintes: les décollages, les atterrissages, les turbulences, la poussée subie..., sont comptées par un système de portes logiques et stockées ensuite en mémoire. Les relevés sont ensuite effectués lors des maintenances, et permettent ainsi d'aiguiller les opérateurs lors de leurs contrôles. Cependant, ces dernières méthodes ne donnent pas directement l'état de la structure. Le document US2002/0163333 A1 décrit un dispositif de surveillance comportant plusieurs capteurs à courants de Foucault, un circuit de traitement relié à ces capteurs pour transformer des mesures des capteurs en données de surveillance, caractérisé en ce que le circuit de traitement est miniaturisé, en ce qu'il est relié par un lien électrique filaire aux capteurs, et en ce qu'il comporte une batterie embarquée, et un moyen radio pour transmettre les données de surveillance à un organe collecteur mobile. Enfin, le document FR2884605 A1 décrit un dispositif similaire de surveillance de la structure d'un véhicule, même si aucune indication n'est donnée quant à la dimension du circuit de traitement ou la longueur de la liaison filaire. Dans l'invention, la solution proposée consiste à utiliser un système autonome, à transmission sans fil, sur batterie et surtout de faible taille pour être placé où cela est souhaité, sans contrainte de taille, dans un moyen de transport, qui réalise en temps réel le diagnostic de l'état de la structure. La méthode mise en oeuvre par ce système autonome consiste à analyser des signaux issus de capteurs par une architecture électronique embarquée, combinant circuits analogiques et numériques. Les algorithmes de traitement étant simplifiés, ils peuvent être embarqués dans un microcontrôleur et le diagnostic peut se faire en temps réel. Les algorithmes étudient notamment l'impédance issue des capteurs et donne l'état et l'évolution des criques. D'autres fonctionnalités déterminent les causes d'apparition des criques, ainsi que leurs évolutions.

Les avantages de l'utilisation de ce système par rapport aux systèmes actuels sont:
- une plateforme électronique modulaire (niveaux interchangeables) permettant le suivi de criques et/ou des contraintes environnementales,
- un suivi continu de criques grâce à une électronique embarquée, autonome et communicant sans fil, permettant un meilleur suivi de l'évolution de la crique,

- une connaissance plus approfondie de l'apparition des criques (les données environnementales sont enregistrées en même temps que l'état de la structure),
- une connaissance plus approfondie de l'évolution des criques (données environnementales associées),
- une connaissance en temps réel (de façon immédiate, sans utilisation de post traitement via un PC) de l'état de la structure (diagnostic), issue de la mesure de l'amplitude,
- une connaissance en temps réel de l'état du capteur (décollé ou non), issue de la mesure de la phase,
- une collecte aisée des informations grâce à une liaison radio,
- une diminution des coûts et du temps de maintenance, évitant ainsi le démontage de certains panneaux lors des maintenances non planifiées,
- une faible consommation de l'équipement, permettant une longue utilisation de ce dernier (1 à 2 ans est la durée visée),
- un faible encombrement de l'équipement (intégration 3 D) pour permettre de le placer dans des endroits difficiles d'accès,
- un fonctionnement autonome, sans raccordement au réseau électrique facilitant son utilisation..

L'invention a donc pour objet un dispositif de surveillance de la structure d'un véhicule tel que défini par la revendication 1. L'invention permet ainsi de pallier les limitations présentées dans les systèmes précédents, à savoir,
- la consommation d'énergie,
- un fort encombrement, et donc une limitation d'accès,
- la présence de grandes liaisons filaires (jusqu'à 50 m),
- la consultation et l'interprétation des données faites uniquement en période de maintenance et non en temps réel,
- le suivi discontinu des structures.

L'invention sera mieux comprise à la lecture de la description qui suit et à l'examen des figures qui l'accompagnent. Celles-ci ne sont présentées qu'à titre indicatif et nullement limitatif de l'invention. Les figures montrent:
- Figures 1a à 1c : un dispositif de surveillance selon l'invention, des variantes de capteurs, et leur mise en place dans un véhicule particulier;
- Figures 2 et 3 des allures de tensions proportionnelles respectivement à une amplitude et à une phase d'un signal délivré par un capteur de l'invention;

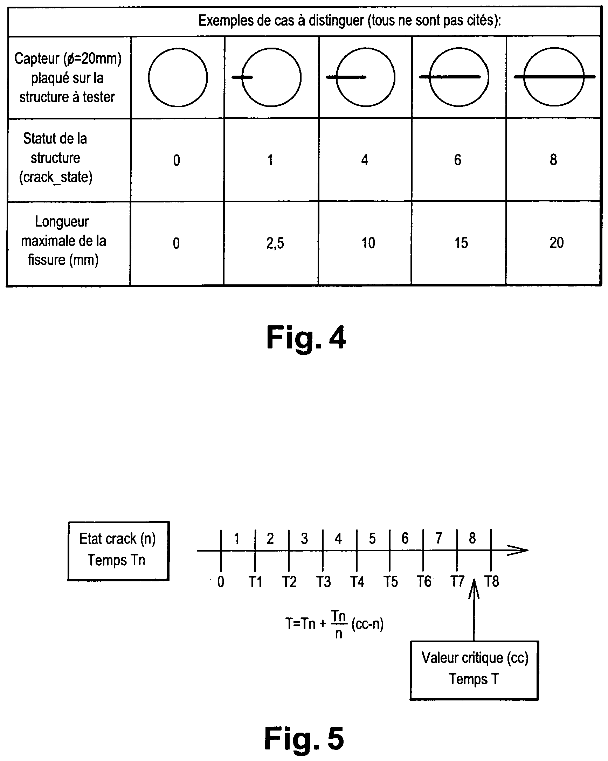

- Figures 4 et 5, des correspondances entre des mesures effectuées par un capteur selon l'invention et une taille d'une crique d'une part et une date d'arrivée à une taille critique d'autre part;
- Figure 6: un exemple d'un algorithme de suivi de crique mis en oeuvre par le circuit de traitement de l'invention.

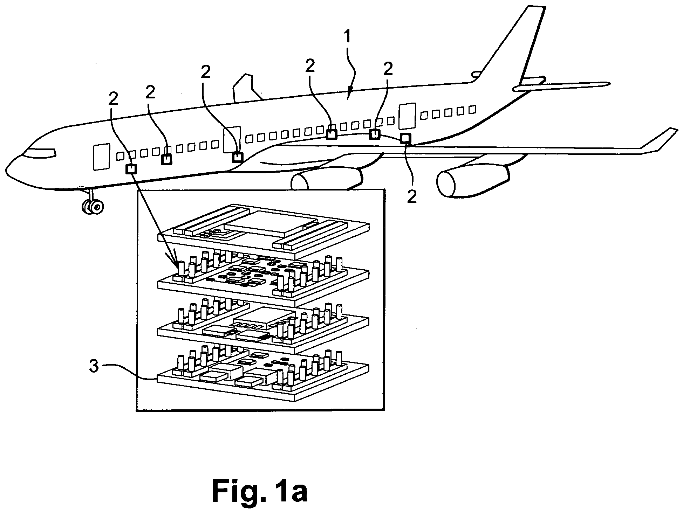

La figure 1a montre un moyen de transport 1, ici un avion, embarquant plusieurs dispositifs de surveillance selon l'invention, réalisés sous forme de plateformes électroniques intégrées, ou noeuds. Chaque noeud, autonome, sans fil et de faible taille, est placé dans un endroit connu comme étant sensible. L'ensemble des plateformes (capteur plus circuit électronique) surveille la structure 1.

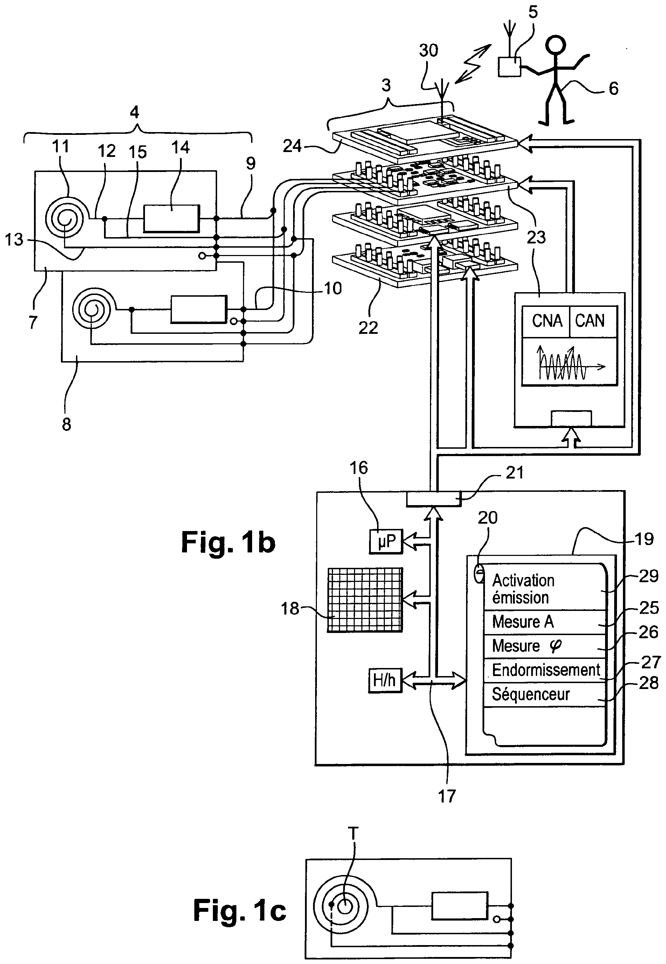

Chaque dispositif comporte, figure 1b, un circuit de traitement 3 relié à un capteur 4. Le circuit 3 transforme les signaux de mesure produits par le capteur 4 en des données de surveillance. Un organe collecteur 5, par exemple porté par un opérateur 6 passant à proximité d'un dispositif 2 de la structure 1, prélève, lors d'une station de préférence immobile du véhicule, les données de surveillance pour une exploitation de maintenance, par exemple pour vérifier l'état de la structure 1, ou pour provoquer le remplacement de certaines pièces.

Selon l'invention, le dispositif 2 est miniaturisé. Dans un exemple il comporte un circuit de traitement 3 dont la taille est comprise dans un cube de 40mm de coté. Une miniaturisation plus forte est encore attendue en fonction de l'évolution des technologies électroniques et d'un routage plus compact. Ce dispositif 2 miniature comporte également un capteur 4. Dans un exemple préféré le capteur 4 est formé d'un couple de deux capteurs 7 et 8, identiques, destinés à être placés, l'un à un endroit à surveiller de la structure 1, l'autre, servant de test, à proximité de cet endroit, dans un endroit de la structure 1 réputé sain. La proximité des deux capteurs est liée à la longueur d'une connexion électrique filaire 9 et 10 qui relie chacun d'eux au circuit 3. Dans un exemple, les liaisons 9 et 10 souples et doubles sont de longueur comprise entre 50mm et 200mm. Les liaisons 9 et 10 peuvent être connectées aux capteurs 7 et 8 et au circuit 3 d'une manière définitive ou par l'intermédiaire de connecteurs détachables. Dans un exemple les liaisons 9 et 10 portent chacun quatre fils. Il n'est toutefois pas nécessaire de disposer de deux capteurs. En effet, le capteur test servant de référence, il est possible de l'omettre en prévoyant par exemple une calibration sur site ou en usine de la réponse du capteur de mesure.

Chaque capteur est formé d'une bobine, ici réalisé de manière préférentielle par une spirale telle que 11 gravée sur un support souple métallisé, par exemple en une couche de polytetrafluoroéthylène métallisé double face. Le support étant double face, deux connexions 12 et 13, ménagées de chaque coté de la bobine, ou sur une même face grâce à un via, sont connectées à des brins conducteurs de la liaison filaire 9. On peut prévoir dans la connexion 12 une résistance 14 d'adaptation en puissance. La connexion 13 est de préférence une connexion de masse, en particulier commune aux deux capteurs 7 et 8, quand il y en a deux. En variante, figure 1c, le ou les capteurs possèdent un trou central T pour pouvoir être monté autour d'un rivet.

A un point intermédiaire entre la bobine 11 et la résistance 14, est connectée une connexion 15 servant à acheminer le signal d'excitation, et donc de mesure, du capteur. Le principe de la mesure est le suivant. Un signal alternatif est produit par le circuit 3 et, acheminé au travers de la résistance 14, se développe dans la structure 1 à l'endroit où le capteur 7 est placé. Si la structure est saine, s'il n'y a pas de crique, l'amplitude du signal correspondra à la présence d'une self et d'une résistance de valeurs connues Si, par contre, la zone sous jacente à la bobine 11 est fendue, s'il y a une crique, la circulation des courants de Foucault est perturbée. Dans ces conditions, l'inductance mutuelle entre le capteur et la source diminue, tandis que la résistance augmente.

La présence d'un capteur test 8 à proximité du capteur de mesure 7 permet de s'affranchir de la calibration, tout en permettant de s'affranchir également des effets de la température. Les signaux mesurés sont alors un rapport ou une différence des amplitudes des signaux mesurés par les capteurs 7 et 8 respectivement. En outre, si un des capteurs 7 ou 8 est détaché de la structure 1, la comparaison de la phase des mesures permet de le détecter immédiatement. En effet, sans inductance mutuelle avec la structure pour l'un d'eux, les signaux injectés dans les capteurs 7 et 8 ne sont alors plus synchrones, il y a alors un décalage de phase.

La figure 1b présente l'intégration à trois dimensions retenue pour le circuit 3, ainsi que les quatre différents niveaux du noeud, l'ensemble formant un cube. Chaque niveau réalise une fonction particulière: une transmission radio, une conversion des signaux issus des capteurs en deux tensions proportionnelles, un traitement des données par microcontrôleur et un stockage des données dans une mémoire, et une alimentation autonome du noeud et les conversions de tensions continues (DC/DC) nécessaires pour alimenter les composants situés sur chaque niveau. Les niveaux sont interchangeables et peuvent être adaptés suivant l'application visée (par exemple des contraintes mécaniques et ou environnementales).

Le circuit 3 comporte ainsi d'une manière schématique, à un niveau, un microprocesseur 16 relié par un bus 17 de données, d'adresses et de commandes, à une mémoire 18 où sont mémorisées des données de surveillance, à une mémoire programme 19 mémorisant un programme 20, à une horloge H/h, éventuellement capable de cadencer le microprocesseur de manière rapide ou lente et à une interface 21 de communication du premier niveau avec les autres niveaux.

L'interface 21 comporte de préférence des broches de connexion traversantes pour relier entre eux les différents niveaux du circuit 3. De ce fait la position de ces derniers est de préférence interchangeable dans le circuit 3 cubique.

Un deuxième niveau 22 du circuit 3, lui aussi relié à l'interface 21, comme tous les autres niveaux, est un niveau de préférence inférieur en ce sens qu'il est destiné à être celui qui reçoit la batterie, ou de préférence une pile capable de fonctionner entre des températures de -60°C et +85°C, et qui est fixé sur la structure, non loin de la structure à surveiller.

Un troisième niveau 23 est en communication entre le niveau du microprocesseur 16-20 et les capteurs 7 et 8 par l'intermédiaire des liaisons 9 et 10. Dans ce niveau 23, les fonctions électroniques effectuées sont des conversions de l'impédance en deux tensions proportionnelles, ainsi que, de préférence, une excitation sinusoïdale des injecteurs reliés aux capteurs.

Un quatrième niveau 24 sert pour la liaison radioélectrique avec l'organe 5.

Dans une application préférée, les différents composants (mémoire, générateur de sinus, conditionnement, radio) communiquent par deux types d'interface série: une interface préférée SPI (Sériai Peripheral Interface) et UART (Emetteur/récepteur asynchrone universel). Le Tableau 1 ci-dessous présente la localisation de chaque fonctionnalité dans le cube :

**Tableau 1**

| Fonction | Numéro du niveau de 1 à 4 en partant du bas) |
|---|---|
| Radio | 4 |
| Conditionnement | 3 |
| Conversions analogique numérique et numérique analogique | 2 |
| Microcontrôleur | 2 |
| Mémoire (par exemple 1 Moctets) | 2 |
| Alimentation, conversion de tension | 1 |
| Capteurs 7, 8 | externes |

Dans le programme 20 on a organisé en sous programmes des tâches 25 à 29. La tâche 25 est une tâche de mesure de l'amplitude du signal délivré par le capteur 7 (et au besoin 8) pour connaître la longueur et la profondeur d'une crique. La mesure de la longueur de la crique sera expliquée plus loin. La modification de la fréquence du signal injecté par l'injecteur permet par ailleurs de connaître la profondeur dans le métal de la crique. Plus la fréquence est élevée, entre 100KHz et 1000KHZ par exemple, moins le signal pénètre dans le métal. Aussi, la réponse donnée par le capteur à différentes fréquences permet de connaître la profondeur de la crique.

La tâche 26 de mesure de phase ϕ entre les signaux des deux capteurs permet de s'assurer que le dispositif est toujours bien au contact de la structure 1.

La tâche 27 est une tâche d'endormissement du processeur 16. Par exemple, pendant plusieurs dizaines de minutes après une mesure, par exemple une heure, le microprocesseur cesse toutes ses fonctions. De préférence dans ce cas, l'horloge H/h de cadencement du microprocesseur passe d'un rythme rapide H à un rythme lent h pour que le comptage de la durée d'endormissement lui même ne pénalise pas la capacité d'autonomie du dispositif. Avec les grandeurs indiquées ci-dessus, pour des piles facilement disponibles, on a estimé que la duré d'autonomie peut être de trois ans. Cette autonomie est à rapprocher des surconsommations rencontrées dans l'état de la technique, qui elles-mêmes imposaient le passage de fils d'alimentation supplémentaires, ce qui grevait le coût et le poids du véhicule.

La tâche 28 représente le système d'exploitation, le séquenceur du microprocesseur 16.

La tâche 29 représente les opérations qui sont lancées lorsqu'un opérateur 6 s'approche du circuit 2. L'organe de collecte 5 émet alors à la demande de l'opérateur 6 un ordre, reçu par une antenne 30 du niveau 24, qui lui-même met en service une émission à destination de l'organe 5 des données mémorisées dans la mémoire 18.

Un intervalle de temps entre chaque prise de mesure peut être configuré dans la tâche 27 via une interface de microordinateur, PC. Les mesures sont ensuite analysées par un algorithme 25-26, contenu dans le circuit 3, et l'état de la structure, symbolisé par un numéro, est enregistré dans la mémoire 18. La date associée à la mesure, et produite par l'horloge H/h, est également sauvegardée. Le diagnostic de la structure est connu immédiatement. Les données sont ensuite récupérées par radio sur un assistant électronique personnel 5, PDA, ou un microordinateur portable. Elles sont affichées dans un éditeur de type tableur, chaque fois que nécessaire, en particulier à chaque maintenance. L'évolution d'une crique est ainsi connue.

Une fonctionnalité supplémentaire permet d'acquérir, en même temps, les conditions environnementales et de les sauvegarder en mémoire. Ainsi, en comparant ces données (état de la structure et données climatiques), les conditions de développement de la fissure sont identifiées. De plus, un seuil critique de longueur de crique peut être intégré au système. Une estimation de la date d'atteinte de cette longueur est automatiquement calculée par l'algorithme. Ce calcul se fonde sur une régression linéaire, à partir des dates enregistrées pour chaque palier franchi. Ainsi, une maintenance peut être planifiée, ou repoussée, si nécessaire (à l'analyse de la longueur de la crique).

L'invention sera utilisée par les compagnies aériennes, maritimes et ferroviaires pour réaliser les maintenances sur leur flotte (avions, hélicoptères, trains, bateaux...). Elle permet de connaître précisément quand une structure est défectueuse (apparition de criques) et l'évolution des défauts. Elle optimise donc les maintenances: le changement d'une pièce peut être avancé ou repoussé, évitant les maintenances non planifiées. De plus, elle limite les coûts et les temps de maintenances, car l'utilisateur doit simplement télécharger les données et inspecter partiellement le véhicule de transport, l'avion. Par ailleurs, les suivis de développement de criques sont améliorés et leurs causes peuvent être identifiées (changement de température, pression, humidité, décollage...).

De préférence, l'ensemble des capteurs forme un réseau et les noeuds communiquent entre eux par liaison radio (par exemple de type Zigbee pour limiter la consommation). Les données, analysées et enregistrées de manière continue, sont collectées régulièrement par une centrale d'acquisition au sol (par exemple, tous les deux ou quatre mois) par radio, lors d'une maintenance de l'avion. Elles donnent directement l'état de la structure testée

Pour limiter la consommation du dispositif, et étant donné le temps moyen de propagation d'une crique, sur 100 000 heures de vol, la valeur par défaut est fixée, dans une configuration préférentielle, à une mesure par heure, le reste du temps, le dispositif est en veille. Les séries de données obtenues sont traitées par un algorithme dans le microcontrôleur 16-20, dès l'acquisition de nouvelles mesures.

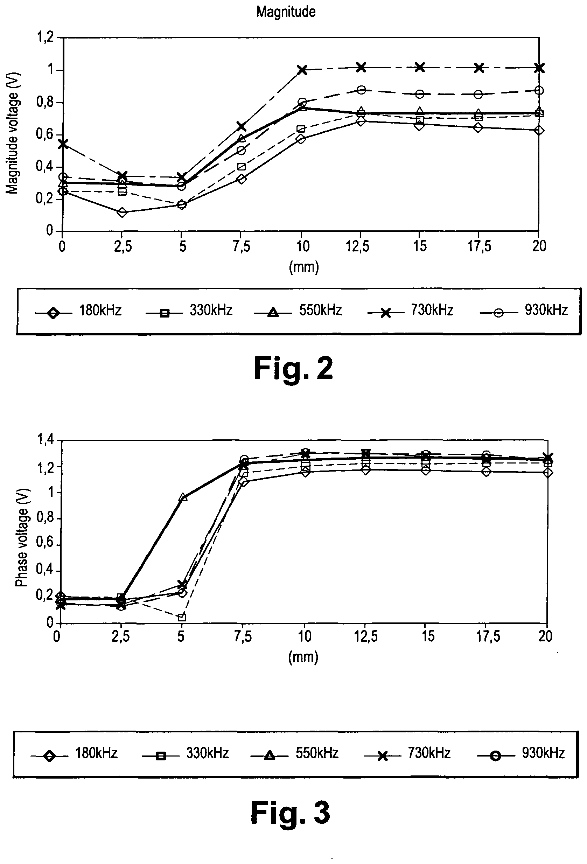

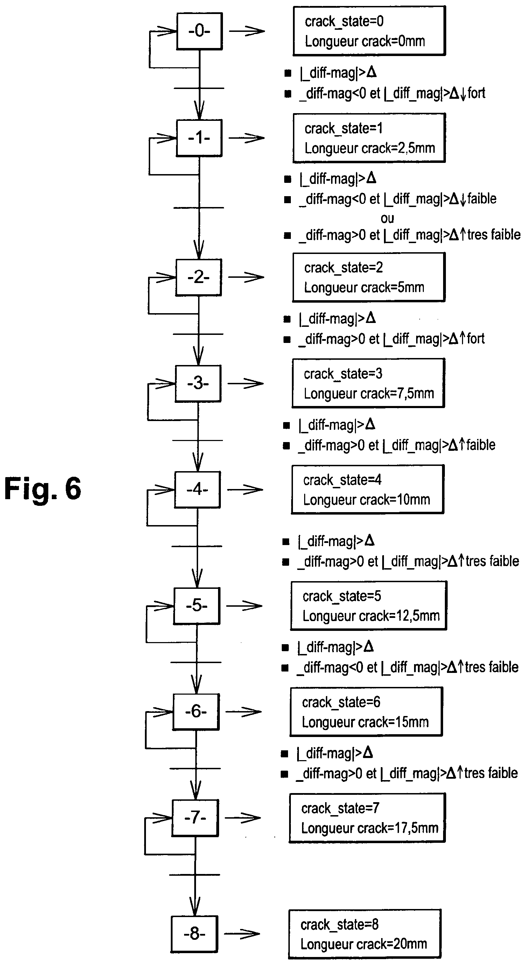

Les figures 2 et 3 présentent les sorties caractéristiques obtenues après le conditionnement. L'évolution des courbes d'amplitude sont d'abord décroissantes puis croissantes avec des pentes plus ou moins abruptes selon la taille de la fissure détectée. L'algorithme présenté en figure 6 se fonde sur cette pente. Cet algorithme situe l'état de la structure (variable nommée _crack_state), et donc la longueur de la fissure résultante, par paliers, numérotés ici de 0 à 8

La surface sensible du capteur à courant de Foucault est celle de la bobine 11. Dans une configuration préférentielle, c'est un cercle de diamètre égal à 20 mm. Cette taille limite la longueur maximale de crique à détecter. Le nombre des différents paliers est également fixé par le diamètre de la surface sensible. Ici, la surface sensible a été subdivisée en 8 parties, soit des longueurs de 2,5 mm. L'algorithme décrit dans la figure 6 peut facilement être adapté pour d'autres surfaces sensibles et pour un nombre de paliers différents.

Le tableau 2 suivant est un descriptif des pentes de la tension caractérisant l'amplitude.

**Tableau 2**

| Palier | Pente positive (↑) | Pente négative (↓) | Pente (+++ : forte; ++ : faible; + : très faible) |
|---|---|---|---|
| 1 | | | +++ |
| 2 | | | ++ |
| 3 | X | | +++ |
| 4 | X | | ++ |
| 5 | x | | + |
| 6 | | X | + |
| 7 | x | | + |
| 8 | | x | + |

L'algorithme utilise principalement l'amplitude de l'impédance de déséquilibre du pont formé par les deux capteurs 7 et 8. Par exemple une différence de 100 mV à 180 KHz est révélatrice d'une crique de longueur 5 mm. La phase est utilisée ici pour savoir si le capteur est correctement collé sur la structure. La première prise de mesure est faite sur une structure saine c'est-à-dire sans crique comme référence. Les deux tensions issues de ces mesures sont stockées dans des variables Mag_n et Phase_n au temps n. Pour les mesures suivantes, les précédentes mesures sont stockées dans les variables caractérisant le temps n-1, Mag_n-1 et Phase_n-1. Les nouvelles valeurs sont stockées dans les variables relatives au temps n. L'algorithme travaille sur la différence d'amplitude de ces deux valeurs (dif_mag) c'est-a dire sur le signe et sur l'écart entre ces dernières. Suivant le signe positif (Δ↑) ou négatif (Δ↓), et la pente plus ou moins abrupte (forte, faible ou très faible) de cette différence l'état caractérisant la structure évolue dans les différents états numérotés de 0 à 8 comme le présente l'algorithme. Le suivi temporel ainsi proposé par l'algorithme de la figure 6 permet ainsi de tenir compte du fait que pour une crique, le signal commence d'abord par décroître avant de croître, avec donc des variations contraires.

Par exemple pour passer de l'état 0 à l'état 1, il faut que la différence d'amplitude soit supérieure d'un seuil Δ↓ fort fixe.

La précision de la mesure est environ 50 mV, c'est pourquoi, toute différence d'amplitude mesurée entre les deux capteurs, inférieure à 50 mV (nommé A dans l'algorithme), ne sera pas prise en compte par ce dernier. Si le capteur est mal collé sur la structure, c'est à dire si les valeurs de phase et d'amplitude dépassent un seuil fixé, un message d'alerte est automatiquement enregistré.

L'algorithme réalise donc, en temps réel, l'analyse de l'état de la structure et stocke ensuite en mémoire la valeur de cet état. La longueur de la crique maximale en découle. Le temps associé est également enregistré, ce qui permet de suivre, précisément, l'évolution de la crique.

Le dispositif possède également une fonction d'acquisition de paramètres environnementaux (température, humidité), ainsi que la pression et les vibrations. Cette fonctionnalité est activée si l'utilisateur veut comparer les différents états de la structure testée avec les paramètres environnementaux enregistrés. Ainsi, les conditions d'apparition et d'évolution des criques pourront être identifiées.

## Revendications

1. Dispositif de surveillance de la structure d'un véhicule comportant un capteur électrique de mesure, un circuit de traitement relié au capteur pour transformer des mesures du capteur en données de surveillance, et un moyen de transmission pour transmettre les données de surveillance à un organe collecteur, le circuit de traitement
- étant miniaturisé, avec une petite taille comprise dans un cube de 40x40x40 mm ou de taille inférieure,
- étant relié par un lien électrique filaire court au capteur, la longueur de liaison étant inférieure à 200mm,
- comportant une batterie embarquée,
- et comportant un moyen radio pour transmettre les données de surveillance à un organe collecteur mobile placé temporairement à proximité ; **caractérisé en ce que** le circuit de traitement est organisé selon une architecture par niveaux, avec un premier niveau d'alimentation électrique placé à la base du circuit près de la structure à surveiller, surmonté d'un deuxième niveau de traitement numérique, lui-même surmonté d'un troisième niveau de conversion des signaux issus des capteurs en deux tensions proportionnelles, le second niveau les convertissant en valeurs numériques, un troisième niveau étant lui-même surmonté d'un quatrième niveau d'émission réception radioélectrique, le quatrième niveau étant placé à la partie supérieure du circuit,
et **en ce que** le capteur est à courants de Foucault et comporte un premier capteur de mesure, placé sur la structure en un endroit à surveiller, et un deuxième capteur test, identique au premier capteur de mesure et placé sur la structure en un endroit sain, pour mesurer par différence de phase entre les signaux produits par les deux capteurs leur parfaite adhésion à la structure à surveiller.

2. Dispositif selon la revendication 1, **caractérisé en ce que** le circuit de traitement comporte une fonction émission du capteur activée lors d'une station immobile du véhicule, par l'organe collecteur.

3. Dispositif selon l'une des revendications 1 à 2, **caractérisé en ce que** le capteur est un capteur de mesure de crique.

4. Dispositif selon l'une des revendications 1 à 3, **caractérisé en ce que** le capteur à courants de Foucault est une bobine en spirale portée par un circuit flexible, dans un exemple de 25x50mm environ, métallisé double face, raccordé au circuit de traitement par une connexion filaire souple double, par exemple de longueur comprise entre 5 et 20 cm, placée à un endroit à surveiller, et associé à un autre capteur de référence monté à proximité de l'endroit à surveiller dans un endroit réputé sain, et **en ce que** le circuit de traitement mesure une différence du signal délivré par un capteur par rapport à l'autre.

5. Dispositif selon l'une des revendications 1 à 4, **caractérisé en ce que** le capteur à courant de Foucault possède une taille de surface donnée, par exemple d'un diamètre de 20mm, et **en ce que** le circuit de traitement comporte des moyens pour quantifier les mesures, par exemple sur 8 niveaux, de façon à mesurer une évolution, par exemple égale à 2,5 mm, d'une taille d'une crique.

6. Dispositif selon l'une des revendications 1 à 5, **caractérisé en ce que** le circuit de traitement comporte une mémoire pour mémoriser les mesures et ou les données de surveillance.

7. Dispositif selon l'une des revendications 1 à 6, **caractérisé en ce que** le circuit de traitement est basse consommation et dans ce but comporte un séquenceur pour réaliser des mesures espacées les unes des autres de plusieurs dizaines de minutes et pour provoquer son endormissement entre les mesures.

8. Dispositif selon l'une des revendications 1 à 7, **caractérisé en ce que** le circuit de traitement comporte une pile capable de fonctionner entre des températures de -60°C et +85°C.

9. Dispositif selon l'une des revendications 1 à 8, **caractérisé en ce que** le circuit de traitement comporte un circuit pour mesurer une date pour mémoriser cette date en correspondance d'une mesure, et un programme pour déduire l'évolution d'une crique malgré des variations contraires du signal de mesure.

10. Dispositif selon l'une des revendications 1 à 9, **caractérisé en ce que** le circuit de traitement comporte un générateur de signal sinusoïdal dont la fréquence est comprise entre 100KHz et 1000KHz pour mesurer la profondeur de la crique, et un séquenceur pour faire varier cette fréquence au cours d'une mesure de manière à explorer la profondeur de la crique.

11. Dispositif selon l'une des revendications 1 à 10, **caractérisé en ce que** le capteur possède un trou au milieu pour être placé autour d'un rivet.

## Patentansprüche

1. Vorrichtung zum Überwachen der Struktur eines Fahrzeugs umfassend einen elektrischen Messsensor, eine mit dem Sensor verbundene Verarbeitungsschaltung zum Umwandeln der Messungen des Sensors in Überwachungsdaten und ein Übertragungsmittel zum Übertragen der Überwachungsdaten zu einem Sammelelement, wobei die Verarbeitungsschaltung
- mit einer kleinen Größe in einem Kubus von 40x40x40 mm oder einer kleineren Größe miniaturisiert ist,
- durch eine kurze Stromkabelverbindung mit dem Sensor verbunden ist, wobei die Verbindungslänge kleiner als 200 mm ist,
- eine Bordbatterie umfasst,
- und ein Funkmittel zum Übertragen der Überwachungsdaten zu einem vorübergehend in der Nähe angeordneten mobilen Sammelelement umfasst;
**dadurch gekennzeichnet, dass** die Verarbeitungsschaltung in einer Architektur in Stufen aufgebaut ist, wobei eine erste Stromversorgungsstufe an der Basis der Schaltung nahe der zu überwachenden Struktur angeordnet ist, der eine zweite Digitalverarbeitungsstufe übergeordnet ist, der wiederum eine dritte Stufe zum Umwandeln der Signals von den Sensoren mit zwei proportionalen Spannungen übergeordnet ist, wobei die zweite Stufe diese in Digitalwerte umwandelt, wobei der dritten Stufe selbst eine vierte Funksende/empfangsstufe übergeordnet ist, wobei die vierte Stufe im oberen Teil der Schaltung angeordnet ist,
und dass der Sensor ein Wirbelstromsensor ist und einen ersten Messensor, angeordnet auf der Struktur an einer zu überwachenden Stelle, und einen zweiten Testsensor, identisch zum ersten Messensor und angeordnet auf der Struktur an einer intakten Stelle, zum Messen durch den Phasenunterschied zwischen den von den zwei Sensoren erzeugten Signalen durch perfektes Haften an der zu überwachenden Struktur umfasst.

2. Vorrichtung nach Anspruch 1, **dadurch gekennzeichnet, dass** die Verarbeitungsschaltung eine Funktion zum Senden des aktivierten Sensors bei einem Stillstand des Fahrzeugs durch das Sammelelement umfasst.

3. Vorrichtung nach einem der Ansprüche 1 bis 2, **dadurch gekennzeichnet, dass** der Sensor ein Rissmesssensor ist.

4. Vorrichtung nach einem der Ansprüche 1 bis 3, **dadurch gekennzeichnet, dass** der Wirbelstromsensor eine von einer flexiblen Schaltung getragene Spiralspule von in einem Beispiel etwa 25x50 mm, doppelseitig metallisiert, durch eine doppelte flexible Kabelverbindung beispielsweise mit einer Länge von 5 bis 20 cm mit der Verarbeitungsschaltung verbunden, angeordnet an einer zu überwachenden Stelle und mit einem in der Nähe der zu überwachenden Stelle an einer als intakt bekannten Stelle weiteren Referenzsensor verbunden, ist, und dass die Verarbeitungsschaltung eine Differenz des von einem Sensor gelieferten Signals im Verhältnis zum anderen misst.

5. Vorrichtung nach einem der Ansprüche 1 bis 4, **dadurch gekennzeichnet, dass** der Wirbelstromsensor eine vorgegebene Flächengröße beispielsweise mit einem Durchmesser von 20 mm aufweist, und dass die Verarbeitungsschaltung Mittel zum Quantifizieren der Messungen beispielsweise auf 8 Stufen zum Messen einer Entwicklung, beispielsweise von 2,5 mm, einer Größe eines Risses umfasst.

6. Vorrichtung nach einem der Ansprüche 1 bis 5, **dadurch gekennzeichnet, dass** die Verarbeitungsschaltung einen Speicher zum Speichern der Messungen und/oder der Überwachungsdaten umfasst.

7. Vorrichtung nach einem der Ansprüche 1 bis 6, **dadurch gekennzeichnet, dass** die Verarbeitungsschaltung wenig Strom verbraucht und hierzu einen Folgeschalter zum Durchführen der Messungen in einem Abstand von Vielfachen von zehn Minuten zueinander und zum Bewirken ihres Abschaltens zwischen den Messungen umfasst.

8. Vorrichtung nach einem der Ansprüche 1 bis 7, **dadurch gekennzeichnet, dass** die Verarbeitungsschaltung eine Batterie umfasst, die bei Temperaturen von -60 bis +85 °C funktioniert.

9. Vorrichtung nach einem der Ansprüche 1 bis 8, **dadurch gekennzeichnet, dass** die Verarbeitungsschaltung eine Schaltung zum Messen eines Datums zum Speichern dieses Datums entsprechend einer Messung und ein Programm zum Ableiten der Entwicklung eines Risses trotz entgegengesetzter Variationen des Messsignals umfasst.

10. Vorrichtung nach einem der Ansprüche 1 bis 9, **dadurch gekennzeichnet, dass** die Verarbeitungsschaltung einen Sinussignalerzeuger, dessen Frequenz zwischen 100 kHz und 1000 kHz liegt, zum Messen der Tiefe des Risses und einen Folgeschalter zum Variieren dieser Frequenz im Laufe einer Messung zum Ermitteln der Tiefe des Risses umfasst.

11. Vorrichtung nach einem der Ansprüche 1 bis 10, **dadurch gekennzeichnet, dass** der Sensor ein Loch in der Mitte zur Anordnung um ein Niet aufweist.

## Claims

1. Device for monitoring the structure of a vehicle, including an electrical measurement sensor, a processing circuit linked to the sensor for converting measurements from the sensor into monitoring data, and a transmission means for transmitting the monitoring data to a collecting unit, the processing circuit
- being miniaturized, having a small size contained within a cube of 40x40x40 mm or of smaller size,
- being linked by a short wired electrical link to the sensor, the linking length being less than 200 mm,
- including an embedded battery,
- and including a radio means for transmitting the monitoring data to a mobile collecting unit temporarily positioned nearby;
**characterized in that** the processing circuit is organized into a level-based architecture, with a first electric power supply level positioned at the base of the circuit close to the structure to be monitored, topped by a second numerical processing level, which is itself topped by a third level for converting the signals coming from the sensors into two proportional voltages, the second level converting them into numerical values, a third level itself being topped by a fourth radio transceiver level, the fourth level being positioned at the upper part of the circuit,
and **in that** the sensor is an eddy current sensor and includes a first measurement sensor, positioned on the structure at a location to be monitored, and a second test sensor, identical to the first measurement sensor and positioned on the structure at a healthy location, for measuring, by way of phase difference between the signals produced by the two sensors, the perfect adherence thereof to the structure to be monitored.

2. Device according to Claim 1, **characterized in that** the processing circuit includes a sensor transmission function that is activated when the vehicle is in a stationary position by the collecting unit.

3. Device according to either of Claims 1 and 2, **characterized in that** the sensor is a crack measurement sensor.

4. Device according to one of Claims 1 to 3, **characterized in that** the eddy current sensor is a wound coil borne by a metallized dual-face flexible circuit board, in one example of around 25x50 mm, connected to the processing circuit via a dual flexible wired connection, for example with a length of between 5 and 20 cm, positioned at a location to be monitored and associated with another reference sensor mounted near the location to be monitored in a location deemed to be healthy, and **in that** the processing circuit measures a difference in the signal delivered by one sensor with respect to the other.

5. Device according to one of Claims 1 to 4, **characterized in that** the eddy current sensor has a given surface size, for example with a diameter of 20 mm, and **in that** the processing circuit includes means for quantifying the measurements, for example over 8 levels, so as to measure a development, for example equal to 2.5 mm, in the size of a crack.

6. Device according to one of Claims 1 to 5, **characterized in that** the processing circuit includes a memory for storing the measurements and/or the monitoring data.

7. Device according to one of Claims 1 to 6, **characterized in that** the processing circuit has low energy consumption and, to this end, includes a sequencer for performing measurements that are spaced apart from one another by several tens of minutes and for putting it into sleep mode between the measurements.

8. Device according to one of Claims 1 to 7, **characterized in that** the processing circuit includes a battery that is capable of operating between temperatures of -60°C and +85°C.

9. Device according to one of Claims 1 to 8, **characterized in that** the processing circuit includes a circuit for measuring a date in order to store this date in correspondence with a measurement, and a program for deducing the development of a crack in spite of contrasting variations in the measurement signal.

10. Device according to one of Claims 1 to 9, **characterized in that** the processing circuit includes a generator of sinusoidal signals, whose frequency is between 100 kHz and 1000 kHz, for measuring the depth of the crack, and a sequencer for varying this frequency over the course of a measurement so as to explore the depth of the crack.

11. Device according to one of Claims 1 to 10, **characterized in that** the sensor has a hole in the middle in order to be positioned about a rivet.
